(19) Europäisches Patentamt
European Patent Office
Office européen des brevets

(11) Publication number: **0 249 561 B1**

(12) **EUROPEAN PATENT SPECIFICATION**

(45) Date of publication of patent specification: **13.05.92**

(51) Int. Cl.⁵: **A61K 9/50**, A61K 47/00, A61K 45/08

(21) Application number: **87401327.9**

(22) Date of filing: **12.06.87**

(54) **Compositions using liposome-encapsulated non-steroidal anti-inflammatory drugs.**

(30) Priority: **12.06.86 US 873584**
**24.11.86 US 934151**
**11.06.87 US 61186**

(43) Date of publication of application:
**16.12.87 Bulletin 87/51**

(45) Publication of the grant of the patent:
**13.05.92 Bulletin 92/20**

(84) Designated Contracting States:
**AT BE CH DE ES FR GB GR IT LI LU NL SE**

(56) References cited:
**EP-A- 0 092 121**
**EP-A- 0 092 453**
**WO-A-84/02076**
**WO-A-85/00751**
**WO-A-85/04578**
**US-A- 4 377 567**
**US-A- 4 378 354**

**CHEMICAL ABSTRACTS, vol. 98, no. 6, 7th January 1983, page 364, abstract no. 40611e Columbus, Ohio, US**

**SCIENCE, vol. 219, 18th March 1983, pages 1327-1329 Washington, DC, US L. LICHTENBERGER et al.: "Role of surface-active phospholipids in gastric cytoprotection"**

**CHEMICAL ABSTRACTS, vol. 99, no. 12, 19th September 1983, page 344, abstract no. 93646x Columbus, Ohio, US Y. MIZUSHIMA et al.: "Antiinflammatory effects of indomethacin ester incorporated in a lipid microsphere"**

(73) Proprietor: **THE LIPOSOME COMPANY, INC.**
**One Research Way Princeton Forrestal Center**
**Princeton, NJ 08540(US)**

(72) Inventor: **Weiner, Alan L.**
**117 Oaklyn Terrace**
**Lawrenceville New Jersey 08648(US)**
Inventor: **Cullis, Pieter R.**
**1329 Walnut Street**
**Vancouver V6J 3R2(CA)**

(74) Representative: **Ahner, Francis et al**
**CABINET REGIMBEAU, 26, avenue Kléber**
**F-75116 Paris(FR)**

**EP 0 249 561 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### FIELD OF INVENTION

The present invention is directed to compositions useful for the treatment of disease states, such as inflammation, pain and fever, said compositions comprising a non-steroidal anti-inflammatory drug which may be encapsulated in a liposome. More particularly, the invention describes methods for reducing toxic side effects related to non-steroidal anti-inflammatory drug, by administering those drugs in liposomes, wherein the composition also includes a glycolipid.

In a preferred embodiment this invention relates to the novel concept of substantially encapsulating nonsteroidal anti-inflammatory drugs or therapeutic agents and analogues and derivatives thereof in gastric resistant liposomes to reduce gastrointestinal irritation associated with the administration of such therapeutic agents to mammals including humans.

The preferred composition described herein provide gastric resistant liposomes that contain encapsulated nonsteroidal anti-inflammatory therapeutic agents and analogues and derivatives thereof. These compositions are primarily free of exogenous nonateroidal anti-inflammatory therapeutic agents previously associated with therapy regimens and particularly with chronic therapy regimens (in excess of about three or four days), thus minimizing gastrointestinal insult.

### BACKGROUND OF THE INVENTION

Recent pharmacological research has disclosed a group of anti-inflammatory therapeutic agents which are not steroidal in character. These agents may generally be classified as carboxylic acids (including salicylates, acetic acids, propionic acids and fenamates), pyrazoles, and oxicams).

While these therapeutic agents have been widely accepted they share a general characteristic of potentially being associated with gastrointestinal irritation and lesions. For example, indomethacin, a widely used agent, which is an acetic acid salt, is known to be associated with gasrointestinal ulcers. See, e.g., the work of Miklos Ghyzy et al. in U.S. Patent Nos. 4,378,354 and 4,421,747. These patents disclose improvements in reducing nonsteroidal anti-inflammatory associated ulceration by admixing nonsteroidal anti-inflammatories with phospholipids. Liposomes are generally known but conditions necessary to avoid or minimize nonsteroidal anti-inflammatory associated gastrointestinal insult have remained unidentified. See, e.g., Science 219:1327-1329 March 19, 1983 "Role of Surface-Active Phospholipids in Gastric Cytoprotection," Lichtenberger et al.

Liposomes are completely closed bilayer membranes containing an entrapped aqueous volume. Liposomes may be unilamellar vesicles (possessing a single membrane bilayer) or multilamellar vesicles (onion-like structures characterized by multiple membrane bilayers, each separated from the next by an aqueous layer). The structure of the resulting membrane bilayer is such that the hydrophobic (non-polar) "tails" of the lipid orient toward the centre of the bilayer while the hydrophilic (polar) "heads" orient towards the aqueous phase.

The original liposome preparation of Bangham et. al. (J. Mol. Biol., 13, 238-252 1965) involves suspending phospholipids in an organic solvent which is then evaporated to dryness leaving a phospholipid film on the reaction vessel. Then an appropriate amount of aqueous phase is added, the mixture is allowed to "swell," and the resulting liposomes which consist of multilamellar vesicles (MLVs) are dispersed by mechanical means, This technique provides the basis for the development of the small sonicated unilamellar vesicles described by Papahadjopoulos et al. (Biochim, Biophys, Acta, 135, 624-638 1967), and large unilamellar vesicles.

Other techniques that are used to prepare vesicles include those that form reverse-phase evaporation vesicles (REV), Papahadjopoulos et al., U.S. Patent No. 4,235,871, stable plurilamellar vesicles (SPLV), Lenk et al., U.S. Patent No. 4,522,803, monophasic vesicles (MPV), Fountain et al., U.S. Patent No. 4,588,578 and freeze and thaw multilamellar vesicles (FATMLV), Bally et al., U.S. Application Serial No. 752,423, filed July 5, 1985, and U.S. Patent Application Serial No. 800,545, filed November 21, 1985 published in WO87/00043

In a liposome-drug delivery system, the medicament is entrapped in the liposome and then administered to the patient to be treated. For example, see Rahman et al., U.S. Patent No. 3,993,754; Sears, U.S. Patent No. 4,145,410; Papahadjopoulos et al., U.S. Patent No. 4,235,871; Schneider, U.S. Patent No. 4,224,179, Lenk, et al., U.S. Patent No, 4,522,803, and Fountain, et al., U.S. Patent No. 4,588,578.

References citing the use of phospholipide to treat gastrointestinal ulceration have appeared in the literature; this effect due to the purported ability of the lipid to restore the stomach's natural protective barrier to irritants. For example, Dial et al., Gastroenterology, 87, 379-385 (1984), suggested the anti-ulcer

activity of bovine milk was due to its concentration of dipalmitoyl phosphatidylcholine. Other studies cite lysolecithin (Clemencon et al., Scad. J, Gastroenterol. Suppl., 19, 116-120, 1984) and lipids isolated from both the fruits of the Melia azedarach plant, and the mussel Perna canaliculus (Al-Khatib, Jpn. J. Pharmacol., 36, 527-533, 1984, and Rainsford et al., Arzneim,-Forsch., 30, 2128-2132, 1980, respectively), as ulceroprotective agents in rats.

Another study assessing membrane damage incurred by sodium dodecyl sulfate cites phosphatidylcholine (Martin et al., J. Pharm. Pharmacol., 33, 754-759, 1981) as a protective agent against such damage. Finally, Lichtenberger et al. (Science, 219, 1327-1328, 1983) studied the ameliorative effects of a liposomal phospholipid suspension composed of 135 ug of dipalmitoyl phosphatidylcholine, and 15 ug each of phosphatidylethanolamine, phosphatidylglycerol, phosphatidylinositol, and sphingomyelin. They postulate the enhanced protection due to formation of an absorbed hydrophobic layer between the gastric epithelium and the luminal contents. Prostaglandins have been cited as protectants against gastric ulcerogenesis and bleeding in laboratory animals and man (Robert et al., Gastroenterology, 77, 433, 1979, and Robert et al., Gastroenterology, 55, 481, 1968), however Lichtenberger et al. (vide infra) determined that prostaglandin synthesis was not required for extrinsic phospholipid-induced gastric protection. However, when rats were dosed with Prostaglandin $E_2$, rat stomach mucosa demonstrated a 2-6 fold increase in the major gastric surface protective surfactant with the greatest enhancements seen in concentrations of phosphatidylethanolamine and phosphatidylcholine.

In addition, Lichtenberger et al. European Pat. Appl. 92121, published October 26, 1983, suggest phospholipid compositions for the treatment of ulcer. An application for similar compositions combined with a prostaglandin, for treatment of gastric and intestinal ulcers is Imagawa et al., European Pat. Appl. 150732, published August 7, 1985. A further reference suggesting anti-ulcer compositions is Amen et al., U.S. Patent No. 4,029,773, for a saccharose, amino acid and triglyceride mixture.

Ghyczy et al., U.S. Patent No. 4,528,193, discloses compositions and methods of treating inflammation comprising phospholipids and non-steroidal anti-inflammatory drugs where the molar ratio is about 1:0.1 to 1:20. The mixture is prepared by co-solubilizing the drug and lipid in organic solvent, followed by removal of the solvent by distillation. Alternatively, the components are co-mixed in water. The solutions so obtained are then lyophilized.

A major plant galactolipid, digalactosyl diglyceride (DGDG) has been used to prepare liposomes. DGDG accounts for about 40% of the total lipid content of higher plant chloroplast and thylakoid membranes (Quinn et al., Prog. Biophys. Molec. Biol., 34, 109-173, 1978). It has been used in studies where photosystems utilizing chlorophylls and cytochromes are reconstituted into liposomes (Sprague et al., J. Cell Biol., 100, 552-557, 1985, and Morschel et al., J. Cell Biol. 97, 301-310, 1983, respectively). Studies involving immunological activity of DGDG in liposomes as measured by complement dependent glucose release (Alving et al., Immunochemistry, 11, 475-481, 1974) and the reactivity of sera from multiple sclerosis patients with DGDG liposomes and its ability to cause complement-mediated lysis of the liposomes (Boggs et al., J. Neurol. Sci., 66, 339-348, 1984) have been performed. DGDG has been suggested as a minor liposome component for the purpose of delivering liposomal-encapsulated drugs to hepatocytes (Geho, U.S. Patent No. 4,377,567).

There is an ongoing need for compositions which can buffer the unwanted gastrointestinal side effects of NSAIDs.

It is an object of this invention to provide a form of nonsteroidal anti-inflammatory therapeutic agent including analogues and derivatives thereof that is administerable with minimized gastrointestinal ulceration or irritation.

It is a particular object of this invention to provide a form of nonsteroidal anti-inflammatory therapeutic agent including analogues and derivatives thereof that is administerable per os and does not cause gastrointestinal ulceration or irritation.

It is a further object of this invention to provide a gastric resistant liposome preparation containing nonsteroidal anti-inflammatory agents and analogues and derivatives thereof within the liposome, yet has limited or no exogenous nonsteroidal anti-inflammatory agent present.

It is another object of this invention to provide a gastric resistant liposome that when administered to an animal releases nonsteroidal anti-inflammatory therapeutic agent in a controlled manner.

## SUMMARY OF THE INVENTION

The present invention presents new and surprisingly nonirritating preparations for administering non-steroidal anti-inflammatory therapeutic agents to mammals including humans. The term nonsteroidal anti-inflammatory therapeutic agents shall be understood to include the analogues and derivatives of such agents.

The invention provides lipsomal compositions as claimed in claims 1 to 10, comprising a therapeutically effective amount of at least one non steroidal anti-inflammatory therapeutic agent primarily encapsulated in a gastric resistant liposome and primarily absent exogenous non steroidal anti-inflammatory therapeutic agent; optionally the composition additionally comprising a pharmaceutically acceptable carrier or diluent.

Particular compositions are provided for non-steroidal anti-inflammatory drugs with a glycolipid. The glycolipid can be a glycosphingolipid or a galactolipid, such as digalactosyl diglyceride. The pharmaceutical composition may be a liposome composition composed of the above-mentioned glycolipids.

In addition, this invention comprises the preparation and use of gastric resistant liposomes containing nonstoroidal anti-inflamatory therapeutic agents, wherein the preparations are primarily free of exogenous nonsteroidal anti-inflamatory therapeutic agents.

Preferred liposomes of this invention are prepared from both unsaturated lipids, saturated lipids and mixtures thereof. Saturated lipids do not contain carbon-carbon double bonds on the long chain fatty acid component of the lipid. Unsaturated lipids can be hydrogenated to obtain saturated lipids with carbon-carbon double bonds removed. Alternatively, saturated lipids can be synthesized using, for example, saturated long chain fatty acids.

Unless otherwise differentiated, the terms saturated and hydrogenated are used synonomously herein to mean a lipid which does not contain a carbon-carbon double bond in its long chain fatty acid portion.

This invention includes a composition comprising a therapeutically effective amount of at least one nonsteroidal anti-inflammatory therapeutic agent primarily encapsulated in a gastric resistant liposome and primarily absent exogenous nonsteroidal anti-inflammatory therapeutic agent. Preferred aspects of the composition are (1) liposomes comprised of egg phosphatidylcholine or digalactosyldiglyceride, (2) liposomes substantially comprised of a lipid which is saturated such as hydrogenated phosphatides such as hydrogenated egg or soy phosphatidylcholine (3) indomethacin as a nonsteroidal anti-inflammatory therapeutic agent, (4) as to the total weight of nonsteroidal anti-inflammatory therapeutic agent, the exogenous percentage thereof is less than 30% with less than 25% being more preferred and less than 5% further preferred, (5) liposomes of substantially equal solute distribution such as monophasic vesicles or frozen and thawed multilammellar vesicles being more preferred while stable plurilamellar vesicles are preferred for liposomes substantially of saturated lipid, and (6) the composition adapted for oral administration. Further included are compositions wherein the nonsteroidal anti-inflammatory therapeutic agent is selected from salicylates, acetic acids, priopionic acids, fenamates, pyrazoles, oxicams and analogues and derivatives thereof.

This invention also includes a pharmaceutical dosage form comprising at least one nonsteroidal anti-inflammatory therapeutic agent primarily encapsulated in a gastric resistant liposome and primarily absent exogenous nonsteroidal anti-inflammatory therapeutic agent in an acceptable pharmaceutical carrier or diluent. Preferred aspects of the pharmaceutical dosage form include that, as to the total weight of nonsteroidal anti-inflammatory therapeutic agent, the exogenous percentage thereof is less than 30% with less than 25% being more preferred and less than 5% further preferred. In the most preferred embodiment the pharmaceutical dosage form is adapted for oral administration, particularly, with unsaturated lipid liposomes, as a monophasic vesicle or a frozen and thawed multilamellar vesicle and further where either vesicle type contains indomethacin. Similarly, the preferred dosage form for liposomes substantially of saturated lipid is the stable plurilamellar vesicle form. Further included are compositions wherein the nonsteroidal anti-inflammatory therapeutic agent is selected from salicylates, acetic acids, priopionic acids, fenamates, pyrazoles, oxicams and analogues and derivatives thereof.

This invention also includes a method for preparing composition for minimizing the gastrointestinal irritation associated with the administration of a therapeutically effective dose of at least one nonsteroidal anti-inflammatory therapeutic agent to a mammal including a human comprising the use of said nonsteroidal anti-inflammatory therapeutic agent to said mammal in the form of a gastric resistant liposome which primarily encapsulates said nonsteroidal anti-inflammatory therapeutic agent and in which exogenous nonsteroidal anti-inflammatory therapeutic agent is primarily absent. Preferred aspects of the method are (1) using preparations wherein as to the total weight of nonsteroidal anti-inflammatory therapeutic agent the exogenous percentage thereof is less than 30% with less than 25% being more preferred and less than 5% further preferred, (2) using liposomes of substantially equal solute distribution particularly monophasic

vesicles and frozen and thawed multilamellar vesicles, and as to liposomes substantially of saturated lipid using stable plurilamellar vesicles and (3) using indomethacin as a nonsteroidal anti-inflammatory therapeutic agent and (4) employing the method in the oral administration of the composition. Further included are compositions wherein the nonsteroidal anti-inflammatory therapeutic agent is selected from salicylates, acetic acids, priopionic acids, fenamates, pyrazoles, oxicams and analogues end derivatives thereof.

## BRIEF DESCRIPTIONS OF THE DRAWING

FIG 1. Fractionation of indomethacin-containing EPC MPV by sucrose gradient centrifugation. Monophasic vesicles constructed with 100 mg of egg phosphatidylcholine, 0.25 uCi of $^{14}$C-indomethacin, and either 15, 17, 19, 21, 23, or 25 mg of indomethacin were loaded onto linear 5-20% sucrose gradients and centrifuged at 288,000 xg for 2.5 hours.

Fig. 2 Comparative plasma levels of therapeutic agent obtained using saturated and unsaturated lipid liposomes.

## DETAILED DESCRIPTION OF THE INVENTION

The following abbreviations will be employed:

SPLV - stable plurilamellar vesicle
MLV - multilamellar vesicle
MGDG - monogalactosyl diglyceride
DGDG - digalactosyl diglyceride
CHS - cholesterol hemisuccinate
HSPC- hydrogenated soy phosphatidylcholine
THS - tocopherol hemisuccinate
MPV - monophasic vesicle
FATMLV - MLVs produced by a freeze and thaw technique
VET - vesicles formed by one or more extrusions through filter apparatus
REV - reverse phase evaporation vesicle
NSAID - nonsteroidal anti-inflammatory drug

The nonsteroidal anti-inflammatory drugs are a highly effective group of drugs, however, their use is limited due to their toxicity. We have found that administering NSAIDs encapsulated in liposomes reduces gastrointestinal side effects including ulceration while maintaining their efficacy.

The nonsteroidal anti-inflammatory therapeutic agents employed in this invention include by way of example:

Carboxylic acids

Salicylates

Acetylsalicylic Acid (ASA)
Salsalate
Diflunisal
Fendosal

Acetic Acids

Indomethacin
Acemetacin
Cinmetacin
Sulindac
Tolmetin
Zomepirac
Diclofenac
Fenclofenac
Isoxepac
Furofenac
Fentiazac

Clidanac
Oxepinac
Fenclorac
Lonazolac
Metiazinic Acid
Clopirac
Amfenac
Benzolfenac
Clometacine
Etodolac
Bumidazone
Clamidoxic Acid

Propinonic Acids

Ibuprofen
Flurbiprofen
Naproxen
Ketoprofen
Fenoprofen
Benoxaprofen
Indoprofen
Pirprofen
Caprofen
Oxaprozin
Pranoprofen
Suprofen
Microprofen
Tioxaprofen
Alminoprofen
Cicloprofen
Tiaprofenic Acid
Furaprofen
Butibufen
Fenbufen
Furobufen
Bucloxic Acid
Protizinic Acid

Fenamates

Mefanamic Acid
Flufenamic Acid
Meclofenamate
Niflumic Acid
Tolfenamic Acid
Flunixin
Clonixin

Pyrazoles

Phenylbutazone and Analogs
Peprazone (Prenasone)
Apazone (Azapropazone)
Trimethazone
Mofebutazone
Kebuzone
Suxibuzone

Oxicams

Piroxicam
Isoxicam
Tenoxicam

Useful in liposome preparation are lipids that result in a bilayer such that a hydrophobic portion of the lipid orients toward the bilayer while a hydrophilic portion orients toward an aqueous phase.

Lipids that may be used in the present invention include glycolipids such as glycosphingolipids and galactolipids such as digalactosyl diglyceride (DGDG) or monogalactosyl diglyceride (MGDG) and DGDG and/or MGDG in combination with phospholipids such as phosphatidylcholine, phosphatidylserine, phosphatidylinositol, or phosphatidylethanolamine and their derivatives and sterol or tocopherol monoesters of diacids, such as cholesterol hemisuccinate and tocopherol hemisuccinate, respectively.

Glycolipids that may be used in forming the vesicles of the invention include glycosphingolipids and galactolipids such as monogalactosyl diglyceride (MGDG) or digalactosyl diglyceride (DGDG) preferably DGDG. DGDG occurs in nature as a plant lipid in chloroplasts and has the structure:

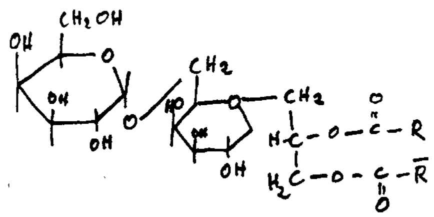

wherein each R substituent is in the $C_{15}$-$C_{17}$ chain range, ca 20% palmitic, 9% oleic, 66% linoleic, the balance being stearic, linolenic, and other fatty acids. (Myhdre, Can. J. Chem., 46, 3071-77, 1968)

The MGDG molecule has a single neutral galactose residue on its polar headgroup. Biophysical studies ($^{13}C$-longitudinal relaxation times) imply that the MGDG headgroup is significantly smaller than that of other lipids such as DGDG and phosphatidylglycerol. Hence, the molecule has a cone shaped structure, with the interactions of the highly unsaturated acyl chains giving it a relatively bulky hydrophobic region. Thus MGDG does not form lamellar structures but forms a hexagonal-II structure under hydration. Mixtures of MGDG with bilayer forming lipids will adopt a bilayer structure at concentrations of MGDG up to about 60 mol %. Higher proportions of MGDG will result in lipidic particles and other nonbilayer structures.

DGDG has an additional galactose unit on the polar headgroup compared to MGDG, thereby giving it a relatively bulky headgroup, but it also has bulky hydrophobic acyl chains. Structurally, DGDG forms cylindrical-shaped structures and induces bilayer organization in membranes. This feature makes DGDG the preferred galactolipid of the two for the formation of liposomes. In combined aqueous dispersions, MGDG and DGDG form mixed lamellar and inverted micelle phases at a 2:1 weight ratio. Studies have been undertaken to form liposomes with MGDG and DGDG combinations using detergent solubilization techniques with Triton X-100. Using this technique, bilayer structures may be formed using both galactolipids only up to MGDG:DGDG weight ratio of 20:80. $Hex_{II}$ tubes formed in preparations using 30% or greater MGDG. Mixtures containing equal weights of MGDG and DGDG produce structures with only traces of bilayer areas containing lipidic particles.

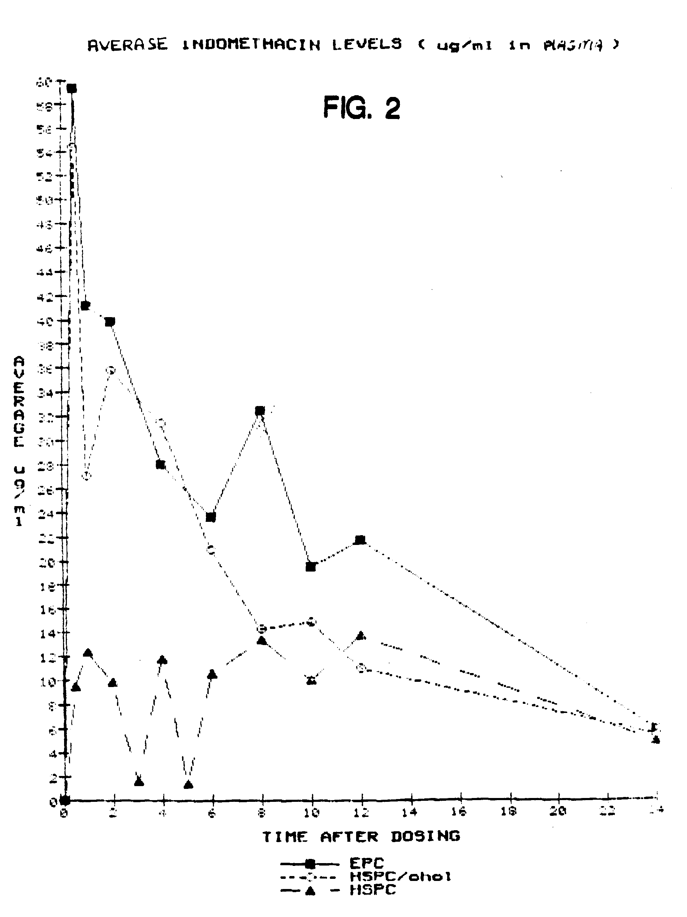

Phospholipids are also useful. Lipids may be utilized alone of in combination. Preferred lipids that offer "gastric resistance" (more fully described below) include the phosphatides, phosphatidylcholine, phosphatidylethanolamine, phosphatidylglycerol, as well as sphingomyelin. Particularly preferred in dosage applications requiring a controlled elaboration of nonsteroidal anti-inflammatory therapeutic agent are liposomes substantially comprised of saturated or hydrogenated lipids, particularly phosphatides and most particularly hydrogenated egg or soy phosphatidylcholine. The controlled elaboration of therapeutic agent provided by liposomes comprised substantially of saturated lipid is distinct in avoiding a peak level of therapeutic agent essentially coincident with administration. This fact can be observed from the blood levels of indomethacin associated with administration of hydrogenated soy phosphatidylcholine orally administered to rats, FIG.2. Peak blood levels have been associated with the contraindications of nonsteroidal anti-inflammatory therapy by some investigators.

The term "substantially" referring to the amount of saturated lipid comprising such a liposome refers to at least about 1:1 saturated lipid to unsaturated lipid based upon the molar ratios of the lipids with about 9:1 saturated to unsaturated being more prefered and essentially all lipid fully saturated being most prefered. Liposomes of saturated lipids may be admixed with other lipids, particularly cholesterol.

Certain lipids are amphipathic only when in the form of a salt and not in the acid state and thus do not form gastric resistant liposomes. Cholesterol and tocopherol hemisuccinates ("CHS" and "THS," respectively) are exemplary of such materials. Upon exposure to conditions common in the gastrointestinal tract, the amphipathic salt form of such materials returns to the aqueous soluble acid form and liposomes based upon these materials rapidly break down. This breakdown occurs within seconds of contact with the low pH associated with the gastric environment. In the practice of a preferred aspect of this invention only gastric resistant liposomes may be utilized. Thus, gastric resistant liposomes may be defined as those liposomes prepared from lipids excluding those lipids that cease to be stable in a bilayer structure upon exposure to pH conditions common in the gastrointestinal tract. Gastric resistant liposomes of improved stability may be made from hydrogenation or saturation of unsaturated lipids.

The liposomes that may be used in the invention include MLVs, small or large unilamellar vesicles (SUVs or LUVs, respectively), VETs and those having equal solute distribution, such as SPLVe, MPVs, and FATMLVs.

A variety of methods may be used to prepare a liposome composition comprising an NSAID and a galactolipid, such as DGDG. In one method, the NSAID is combined with the lipid in organic solvent, the solution rotary evaporated to a thin film and finally, the film hydrated with an aqueous medium such as aqueous buffer, forming liposomes. Such a procedure forms MLVs.

Another method for preparing NSAID-galactolipid liposomes is to combine the NSAID with the galactolipid in organic solvent, rotary evaporate the solution to a thin file, and dissolve the dried film in ethanol to which has been added an aliquot of an aqueous medium such as aqueous buffer. This solution is then rotary evaporated to a thin film, and the film then hydrated with aqueous medium, forming liposomes. Such a procedure forms MPVs.

Yet another method for preparing NSAID-galactolipid liposomes is to combine the NSAID with the galactolipid in organic solvent, rotary evaporate the solution to a thin film, and resuspend the film in diethyl ether. A small aliquot of an aqueous medium such as aqueous buffer is then added to the organic solvent solution, and this solution is dried under nitrogen gas to a paste while sonicating in a bath sonicator. The paste is then hydrated with an aqueous medium, forming liposomes. Such a procedure forms SPLVs.

A further method for preparing NSAID-galactolipid liposomes is to form MLVs as described above, then subject these MLVs to a number of freeze and thaw cycles. Such cycles are carried out by first rapidly cooling the MLV suspension to obtain a frozen lipid-aqueous medium mixture, and then warming the mixture. The freezing and warming steps are preferably performed at least about five times. Such vesicles have an equal solute distribution and are known as FATMLVs.

Yet another method for preparing NSAID-galactolipid liposomes is to form MLVs as described above and extrude these liposomes through a filter under pressues of about 700 psi. Such resulting vesicles are known as VETs and can be prepared according to the procedures of Cullis et al., U.S. Patent Application Serial No. 788,017, filed October 16, 1985.

Within the class of useful liposomes is a preferred subclass of liposomes characterized in having solute distribution substantially equal to the solute distribution environment in which prepared. The subclass may be defined as stable plurilamellar vesicles (SPLV) as defined in U.S. Patent No. 4,522,803 to Lenk et al. and further includes both monophasic vesicles described in U.S. Patent No. 4,588, 578 to Fountain et al. and frozen and thawed multilamellar vesicles (FATMLVs) as described in "Solute Distributions and Trapping Efficiencies Observed in Freeze-Thawed Multilamellar Vesicles" Mayer et al. <u>Biochimica et Biophysica Acta</u> 817:193-196 (1985). Liposomes specifically prepared by the process of U.S. Patent Ser. No. 4,522,803 are referred to as stable vesicles. It is believed that the particular stability of the SPLV liposomes arises from the low energy state attendant to solute equilibrium.

Both CHS- and THS-containing vesicles may generally be prepared by any method known to the art for preparing vesicles. In particular, see the procedures of the copending patent applications of Janoff et al., U.S. Patent Application Serial No. 721,630, entitled "Steroidal Liposomes," filed April 10, 1985, Janoff et al., U.S. Patent Application Serial No. 773,429 entitled "Steroidal Liposomes," filed September 10, 1985, and Janoff et al., Serial No. 786,740, entitled "Alpha-Tocopherol Based Vesicles," filed October 15, 1985, respectively. According to these procedures, the powdered forms of sterol or tocopherol monoesters of diacids, such as CHS or THS are added to an aliquot of aqueous buffer, and vortexed to fully suspend the dispersions forming MLVs. The dispersions are then sonicated in a water bath for several hours forming SUVs, and the drug powders added directly to these sonicated vesicles, and vortexed to fully disperse.

Another technique for forming MGDG/DGDG liposomes is to solubilize both lipids below 20°C in a fluorinated hydrocarbon (such as Freon-22) below the boiling point of the fluorinated hydrocarbon. An aqueous medium is then added forming an emulsion. The emulsion is then warmed above the boiling point of the fluorinated hydrocarbon (e.g. 20°C), to remove the fluorinated hydrocarbon, resulting in liposome formation.

In combined dispersions of MGDC end DGDG used to form reverse phase evaporation vesicles (REVs), bilayers accommodate slightly greater amounts of MGDG before major surface irregularities appear, as compared to the detergent dialysis technique. Liposomes formed using a 40:60 weight ratio of MGDG:DGDG contain hex-II tubular arrays in bilayer vesicles. Clumping of the lipids in the aqueous phase does not occur until 70% MGDG is reached. Both MGDG and DGDG may be successfully combined with other lipids such as phospholipids, sterols such as cholesterol esters, or phenols such as tocopherols to form liposomes.

A liposome preparation can also be composed of a combination of CHS and THS, or other organic acid derivatives of a sterol and a tocopherol.

Where necessary, as in the SPLV and MPV procedures, organic solvents may be used to solubilize the lipid during vesicle preparation. Suitable organic solvents are those with a variety of polarities and dielectric properties, including chloroform, acetone, methylene chloride, diethyl and petroleum ethers, and mixtures of chloroform and methanol.

Liposomes entrap an aqueous medium which is enclosed by the lipid bilayers. The aqueous medium can be for example, water or water containing a dissolved salt or buffer. Examples of such salts or buffers can be sodium chloride and phosphate buffered saline (PBS). Other buffers include Tris-HCl (tris-(hydroxymethyl)-aminomethane hydrochloride), and HEPES (N-2-hydroxyethyl piperazine-N′-2-ethane sulfonic acid). Buffers may be in the pH range of between about 5.0 and about 9.5. In the preferred embodiment, the preparations are hydrated with phosphate buffered saline (PBS) at pH of between about 5.0 and 9.5, preferably about pH 7.4. In the case of CHS- and THS-containing vesicles which employed the Tris salt forms of CHS and THS, a Tris/HCl buffer at pH of about 7.4 was used.

The SPLV Process comprises forming a solution of at least one gastric resistant lipid in at least one organic solvent plus a first aqueous component containing at least one nonsteroidal anti-inflammatory therapeutic agent. Sufficient aqueous component to form a monophase is utilized. The next step is evaporating organic solvent from the monophase at temperature and pressure which maintains and facilitates evaporation until a film forms. The next step is adding a second aqueous component to the film and agitating the second aqueous component with the film in order to resuspend the film and to form lipid vesicles. The resulting material may be washed of exogenous nonsteroidal anti-inflammatory therapeutic agent as required. This method is further described in Example 4 employing indomethacin but it is to be understood to apply similarly to other nonsteroidal anti-inflammatory therapeutic agents as well.

The preferred gastric resistant liposomes of this invention containing a nonsteroidal anti-inflammatory therapeutic agent or combinations of such agents are deemed to primarily free of exogenous nonsteroidal anti-inflammatory therapeutic agents when such exogenous material comprises less than about 30% of the total weight of nonsteroidal anti-inflammatory therapeutic agent or agents present. In the preferred preparation less than about 25% of total nonsteroidal anti-inflammatory therapeutic agent is exogenous. The threshold dosage of nonsteroidal anti-inflammatory therapeutic agents causing gastrointestinal irritation and gross gastrointestinal damage (such as ulceration) will vary with a number of factors including the nonsteroidal anti-inflammatory therapeutic agent, the method of administration, the dosage and duration, the mammal and its presenting condition. In general, the most preferred preparations have the least exogenous nonsteroidal anti-inflammatory therapeutic agent and these preparations characteristically have exogenous nonsteroidal anti-inflammatory therapeutic agent of less than about 5% of the total weight of nonsteroidal anti-inflammatory therapeutic agent or agents present and some less than 1%. A further consideration in use of this invention will be the half-life of the nonsteroidal anti-inflammatory therapeutic agent in the blood. While not being bound to any particular theory, it is thought that the nonsteroidal anti-inflammatory therapeutic agents with longer half-lives have a greater propensity to cause gastrointestinal insult. Of course, this factor may be counter-balanced by other factors such as those noted above. Indomethacin, tolmetin, ibuprofen, diclofenac, fenoprofen, acetylsalicylic, and meclofenamate have generally been shown by others to have blood level half-lives under four hours. Mefenamic acid and flurbiprofen have generally been shown by others to have blood level half-lives of about four and six hours respectively. Naproxen, diflunisal, sulindac, piroxicam and phenylbutazone have generally been shown by others to have half-lives from over ten hours to three days. The degree of amelioration of gastrointestinal irritation by the product

and method of this invention is anticipated to vary as to each nonsteroidal anti-inflammatory therapeutic agent but will likely be more pronounced as to nonsteroidal anti-inflammatory therapeutic agents with relatively shorter half-lives.

It has now been determined that liposomal encapsulated nonsteroidal anti-inflammatory therapeutic agent, primarily free of exogenous nonsteroidal anti-inflammatory therapeutic agent, caused considerably less gastrointestinal insult than the administration of equivalent doses of lipid and nonsteroidal anti-inflammatory therapeutic agent as mere admixtures coadminstered. The latter condition clearly comprises the limiting situation wherein virtually all of the nonsteroidal anti-inflammatory therapeutic agent is exogenous.

In preparing gastric resistant MPV liposomes of this invention, an aliquot each of lipid and nonsteroidal anti-inflammatory therapeutic agent are codissolved into absolute ethanol at ratios preferably exceeding about 6:1 by weight (lipid:nonsteroidal anti-inflammatory therapeutic agent). Depending upon various conditions including both the solubility of the nonsteroidal anti-inflammatory therapeutic agent and lipid ratios from generally about 1 ml ethanol per 100 mg lipid may be used. For convenience in the laboratory 5 ml of ethanol is preferred per 200 mg lipid.

An aqueous component is added to the ethanol organic solvent in a ratio with the ethanol of from about 25:1 to about 1:1 (solvent:aqueous). The resulting monophase is placed under rotoevaporation and at reduced pressure until a film forms. The film may be rehydrated to any desired concentration although 200 mg lipid per ml aqueous solution is preferred. This method may be utilized with salicylates, acetic acids, propionic acids, fenamates, pyrazoles, oxicams and analogues and derivatives thereof.

As each nonsteroidal anti-inflammatory therapeutic agent will present a particular set of physical characteristics including a range of solubilities, liposomal entrapment ranges, and desired dosages, a preferred test procedure for determining entrapped/exogenous nonsteroidal anti-inflammatory therapeutic agent is presented in Example 6, but other suitable test procedures are also well known in the art and are useful.

To determine the relative amounts of liposomal entrapped exogenous nonsteroidal anti-inflammatory therapeutic agent a number of procedures well known to those skilled in the art may be used. A method of radiolabeling a sucrose gradient centrifugation was employed.

In the radiolabel procedure radiolabeled nonsteroidal anti-inflammatory is supplemented into the lipid solvent and a small amount of unlabeled aqueous material is thereafter also added. The solvent is then removed by any convenient method such as rotoevaporation. For ethanol, rotoevaporation is conveniently performed at elevated temperature of about 50°C and reduced pressure such as from about 1 to 100 mmHg.

Those skilled in the art will clearly understand the appropriate reaction conditions for other solvents and nonsteroidal anti-inflammatory therapeutic agents.

After solvent removal, the material is resuspended in suitable aqueous solution such as saline.

The resulting liposomes may then be examined for drug loading. This is done by separating liposomes from nonliposomal material by any separating means. Sucrose gradient separation is one such means.

If a sucrose column is used, the column is centrifuged and thereafter fractions collect and counted in a scintillation counter. Radiolabel at the bottom of such column represents exogenous nonsteroidal anti-inflammatory and the fraction at the top represents liposomally encapsulated nonsteroidal anti-inflammatory therapeutic agents. The percentages exogenous and encapsulated may then be calculated.

Results of tests of the egg phosphotidylcholine (EPC)/indomethacin liposomal preparations made by the method of Example 4 which contain increasing levels of indomethacin demonstrate that at weight ratios below approximately 6:1 phospholipid:drug respectively, high density exogenous material containing nonsteroidal anti-inflammatory crystals becomes apparent (FIG. 1). At lower ratios the indomethacin sediments entirely with liposomal structures at the top of the 5% sucrose gradient. Therefore, in all subsequent studies higher phospholipid/indomethacin ratios of about 6:1 by weight or about 2.6:1 molar ratios were employed to insure complete encapsulation of drug with the corresponding primary absence of exogenous nonsteroidal anti-inflammatory drug. Ratios of about 50:1 by weight are also prefered with ratios of 20:1 being most preferred.

It is to be understood that each nonsteroidal anti-inflammatory therapeutic agent will have a particular optimal lipid:drug molar and weight ratios in preparations primarily absent exogenous therapeutic agent. Simple testing via sucrose gradient or other appropriate tests will delimit the lipid:drug ratios appropriate for any reaction conditions to produce liposome preparations primarily absent of exogenous nonsteroidal anti-inflammatory therapeutic agents. So long as an effective liposomally encapsulated therapeutic dosage may be administered while exogenous nonsteroidal anti-inflammatory therapeutic agent is primarily absent by not being present at levels greater than about 30% by weight ulceration and gastric irritation will be

substantially reduced. This limitation as to exogenous nonsteroidal anti-inflammatory is a critical limitation of this invention. A therapeutically effective dosage of nonsteroidal anti-inflammatory therapeutic agent is understood to be that dosage at which the desired physiological response is exhibited.

As to indomethacin in the preferred embodiment no more than about 25% exogenous dosages administered at about 0.5 to 4 mg/kg are utilized. In general, the more limited the exogenous nonsteroidal anti-inflammatory therapeutic agent the less prevalent gastrointestinal irritation and gross gastrointestinal insult. Each nonsteroidal anti-inflammatory therapeutic agent will present a different efficiency of capture within particular liposomes. Where liposomal capture is insufficient to avoid excessive exogenous non-steroidal anti-inflammatory therapeutic agent, liposome preparations may be washed.

The liposome preparations of this invention may be washed of excess exogenous nonsteroidal anti-inflammatory therapeutic agent by centrifugation of the preparation for sufficient time and at sufficient velocity to cause liposomes to sediment but not to lose integrity. The supernatant is decanted and the liposomes are resuspended in saline or other aqueous media. This procedure may be repeated until sufficient exogenous nonsteroidal anti-inflammatory therapeutic agent is eliminated.

Other means of removing excess exogenous nonsteroidal anti-inflammatory therapeutic agents such as dialysis or gel filtrations are suitable and well known in the art.

The compositions and methods of this invention are useful in reducing the adverse reactions to mammals including humans arising from treatment with nonsteroidal anti-inflammatory therapeutic agent. Some measure of this affect is made by acute and chronic ulceration tests as described below.

Gastric ulcers in rats (Wistar) can be acutely induced by oral or subcutaneous administration of nonsteroidal anti-inflammatory therapeutic agents such as indomathacin to animals previously starved for about 18-24 hours. For oral studies, it is convenient to use indomethacin but other nonsteroidal anti-inflammatory therapeutic agent can be similarly employed. This model is also useful in examining other nonsteroidal anti-inflammatory therapeutic agents and other mammals. Simply varying doses will induct gastric ulcers for other agents and mammals in this model. Unlike gastric ulcer protocols, it has been shown that for nonsteroidal anti-inflammatory effects such as indomethacin-induced intestinal lesions to occur, animals must be allowed free acces to food and water during the study.

The acute gastric ulcerative effects observed from administration P.O. to rats of various dosages of indomethacin in PEG-400 compared to indomethacin encapsulated within liposomes that were not gastric resistant ($CHS_t$ and $THS_t$) ("t" denotes the tris salt) and in gastric resistant liposomes (egg phosphatidylcholine MPVs) are illustrated in Table 3. Substantial dose responsive ulceration occured when drug was completely solubilized into PEG-400 vehicle. An average 25mm total ulcer length was obtained at the top dosage of 10 mg indomathacin/kg body weight. In individual experiments this number has reached a high as 45 mm. In liposome preparations indomethacin was fully dissolved into the membrane; and, unlike PEG-400 vehicle, aqueous solutions of gastric resistant indomethacin-EPC MPVs which primarily did not carry exogenous drug were capable of imparting complete protection at the lowest dosage of drug and over 75% protection at higher dosages in chronic administration. Indomethacin-$CHS_t$ liposomes or indomethacin-$THS_t$ liposomes, both sensitive to acid conditions in the GI tract, did not provide as significant gastric protection at the highest dose of drug. Lipids such as $CHS_t$ liposomes or indomethacin-$THS_t$ liposomes, both sensitive to acid conditions in the GI tract, did not provide as significant gastric protection at the highest dose of drug. Lipids such as $CHS_t$ (cholesterol hemisuccinate tris) and $THS_t$ (tocopherol hemisuccinate tris) that typify the class of lipids that are not gastric resistant.

As seen from Table 4, substantial intestinal ulceration is evident following repeated administration of indomethacin in PEG-400. Significant inhibition of ulcer formation is evident at 4 mg/kg oral dosage of drug when EPC MPV encapsulated is administered over the same 4-day schedule. As was observed in acute gastric ulcer experiments, a lack of protective action is also noted in the intestinal model system when indomethacin is incorporated into either $CHS_t$ or $THS_t$ liposomes.

The protection afforded by gastric resistant liposomal (MPV) encapsulation of drug is clearly highlighted in comparison to drug in PEG-400 when intestinal lesions are quantitated after chronic dosing (Table 4). Table 4 further illustrates that protection from ulceration afforded by saturated lipid gastric resistant liposomes is at least equivalent to unhydrogenated liposomes.

The "controlled release" of the encapsulated nonsteroidal anti-inflammatory therapeutic agent of such liposomes substantially comprised of saturated lipid is disclosed in FIG. 2. The "controlled release" pattern of hydrogenated soy phosphatidylcholine is of such level that, via oral dosage, some liposomes will be excreted prior to delivery of all therapeutic agent to the receiving animal. This factor must, of course, be taken into account when selecting the dosage to be adminstered such that sufficient therapeutic agent will be elaborated, prior to excretion of the liposomes, to yield the desired availability of therapeutic agent in the gastrointestinal tract for uptake by the animal.

Each nonsteroidal anti-inflammatory therapeutic agent will have a dose response curve below which chronic administration of therapeutic agent produces no gross damage such as ulceration and above which liposomal encapsulation and primary absence of exogenous therapeutic agent will not afford complete protection. However, this invention is directed to facilitate administration and particularly chronic administration (about 3 or 4 day or longer) of nonsteroidal anti-inflammatory therapeutic agent by reducing gastrointestinal irritation, and by avoiding or reducing ulceration. For indomethacin daily oral dosages below about 2 mg/kg generally produce no gross ulceration but may produce gastrointestinal irritation. Notwithstanding this general case, oral human doses of indomethacin used in this invention are substantially similar to those used in unencapsulated preparations and are usually about 1 mg/kg. These doses may produce gastrointestinal ulceration in humans. The dosages referred to herein are in terms of therapeutic agent that will be available for uptake after release from the encapsulating liposomes. In the case of liposomes substantially comprised of hydrogenated lipid, the liposome may be excreted prior to full release of therapeutic agent. This factor must be taken into account in selecting the dosage to be administered.

Chronic administration of nonsteroidal anti-inflammatory therapeutic agent is a well known method of use of such agents. Chronic administration, for example in arthritis treatment, may be from as short a period as about 3 or 4 days or for as long as for the life of the recipient.

In pharmaceutical preparations, liposomes containing nonsteroidal anti-inflammatory therapeutic agent are suspended in an acceptable pharmaceutical diluent or carriers such as water or saline or other suitable carriers or diluents. A typical preparation comprises from about 1 to 10 ml of aqueous solution containing a therapeutically effective dose. A typical liposome containing indomethacin preparation is about 25 mg indomethacin at least 70% liposomally encapsulated in about 5 or 6 ml aqueous media.

NSAIDs are generally lipophilic, and partition within the lipid portion of the liposome which in turn may be in association with a suitable pharmaceutical carrier. The proportional ratio of active ingredient to carrier will naturally depend on the chemical nature, solubility, and stability of the active ingredient, as well as the dosage contemplated. For the oral mode of administration, an NSAID-liposome composition of this invention can be used in the form of tablets, capsules, lozenges, troches, powders, syrups, elixirs, aqueous solutions and suspensions, and the like. In the case of tablets, carriers which can be used include lactose, sodium citrate, and salts of phosphoric acid. Various disintegrants such as starch, and lubricating agents such as magnesium stearate, sodium lauryl sulfate and talc, are commonly used in tablets. For oral administration in capsule form, useful diluents are lactose and high molecular weight polyethylene glycols. When aqueous suspensions are required for oral use, certain sweetening and/or flavoring agents can be added. For parenteral administration or injection via intravenous, intraperitoneal, intramuscular, subcutaneous, intra-aural or intra- mammary route sterile solutions of the NSAID-liposome composition are prepared and the pH of the solutions are suitably adjusted and buffered. For intravenous use, the total concentration of solutes should be controlled to render the preparation isotonic.

In another example of their use, vesicle-entrapped compounds may be incorporated into a broad range of topical dosage forms including but not limited to gels, oils, emulsions and the like. For instance, the suspension containing the entrapped compound may be added to the aqueous phase as an ingredient in any type of liposome preparation (e.g., SPLVs, MPVs, FATMLVs, MLVs, SUVs, LUVs, REVs and others). This allows the entrapment of the water-insoluble compound in the phospholipid liposomes. Such preparations may be administered as topical creams, pastes, ointments, gels, lotions and the like for direct application to the inflamed area.

NSAIDs are generally administered to humans in dosages ranging from about 20 mg to about 3200 mg a day depending on the NSAID. For example, indomethacin dosage ranges from 50-200 mg/day. Ibuprofen dosage ranges from 1200-3200 mg/day. The actual dosages should generally be determined by a physician. Similarly, other mammals such as horses may be administered these compounds in dosage ranges of 2 mg/kg/day - 800 mg/day (regardless of weight). For example, naproxen dosage for horses is 10 mg/kg/day in two divided dosages when given by oral route of administration. The prescribing physician or veterinarian will ultimately determine the appropriate dose for a given subject, and this can be expected to vary according to the age, weight and response of the individual subject, as well as the nature and severity of the subjects symptoms.

The liposomes of the present invention may be dehydrated, thereby enabling storage for extended periods of time until use. Standard freeze-drying equipment or equivalent apparatus may be used to dehydrate the liposomes. Liposomes may also be dehydrated simply by placing them under reduced pressure. Alternatively, the liposomes and their surrounding medium can be frozen in liquid nitrogen prior to dehydration. Dehydration with prior freezing may include the presence of one or more protective sugars in the preparation, according to the process of Janoff et al., U.S. No 4880635 filed July 26, 1985, entitled "Dehydrated Liposomes." Examples of protective sugars that may be used include but are not limited to

trehalose, maltose, sucrose, glucose, lactose and dextran. Alternatively, multilamellar vesicles may be dehydrated with prior freezing without protective sugars. "When the dehydrated liposomes are to be used, rehydration is accomplished by simply adding an aqueous solution, e.g., distilled water, to the liposomes and allowing them to rehydrate.

The liposomes of the present invention may also be remote loaded with ionizable agents according to the disclosure of Bally et al., U.S. Application Serial No. 749,161, filed June 26, 1985, entitled "Encapsulation of Antineoplastic Agents in Liposomes," Published in WO86/01102. In this procedure, a transmembrane potential is created across the bilayers of the liposomes during formation, and the ionizable agent is loaded into the liposomes by means of the transmembrane potential. This potential is generated by creating a concentration gradient for one or more charged species (e.g., $Na^+$, $K^+$ and/or $H^+$) across the liposome membranes. The concentration gradient is created by producing liposomes having different internal and external media, i.e., internal and external media having different concentrations of one or more charged species. The liposomes may be dehydrated prior to or following loading with agent.

The present invention decreases the ulcerogenic effect of NSAIDs, and may improve the efficacy of such drugs. In addition, such protection may be afforded by the liposomes of the present invention against ulcers produced by stress or alcohol consumption.

In the ulcer protection embodiment of the present invention, ulcerogenic activity of free indomethacin was compared to that of liposome-entrapped indomethacin as well as liposomes substantially free of exogenous indomethacin.

In the anti-inflammatory bioactivity embodiment of the present invention, efficacy of the liposome-drug preparations was measured in some embodiments by the edema intensity of a paw previously injected with an edema producing amount of carageenan.

Reduction of edema following administration of free NSAID such as indomethacin was compared to that following treatment with liposome-entrapped indomethacin.

As may be seen from the foregoing description, therapeutically effective doses of nonsteroidal anti-inflammatory therapeutic agents may be encapsulated in gastric resistant liposomes yet be substantially reduced in gastrointestinal irritation usually associated with nonsteroidal anti-inflammatory therapeutic agent regimens. The reduction in gastric irritation is most marked in chronic administration of nonsteroidal anti-inflammatory therapeutic agent in excess of about three or four days.

Therapeutic regimens will vary considerably based in part upon the mammal being medicated, the condition being treated, and the particular nonsteroidal anti-inflammatory being utilized and whether or not the liposomes will fully release the therapeutic agent prior to excretion. While no exact limits may be placed upon nonsteroidal anti-inflammatory therapeutic regimens of treatment the regimens will often extend from about days to years with doses about 3 times per day of nonsteroidal anti-inflammatory therapeutic agents from about 0.1 to 10 mg/kg (as released). The gastric resistant liposomes of this invention are most conveniently administered orally suspended in any suitable pharmaceutical carrier as an oral medication, however without limitation subcutaneous, intravenous, and intraperitoneal administration and other known methods of adminstration are also contemplated. However, notwithstanding these general parameters, each therapeutic regimen may be individually determined by a physician in view of many factors including age, physical condition of patient and condition being treated. Further, while ulceration or irritation may not be fully avoided, it will be reduced.

PREPARATORY STEP 1

LIPID PURIFICATION

Digalactosyl diglyceride (DGDG) was obtained commercially or was prepared from fresh spinach leaves according to the following procedure:

One hundred grams of spinach leaves were chopped into 1 cm pieces and placed in a Waring blender with 300 ml of isopropanol at 70-80 °C. the mixture was blended on high speed for two minutes. The resulting slurry was filtered through two layers of Whatman #1 filter paper and the residue washed with 200 ml of the hot isopropanol. The resulting filter cake was placed in the blender with 200 ml chloroform:isopropanol (1:1 v/v) and blended as above. The resulting homogenate was filtered as above and the residue washed with 200 ml of chloroform:isopropanol (1:1 v/v) and then 200 ml chloroform. The filtrate was rotoevaporated in vacuo to a lipid film. The film was then dissolved in 200 ml chloroform, and the solution washed three times with 100 ml of 1% (weight/volume) sodium chloride aqueous solution in a separatory funnel. The organic phase was separated and 5 ml benzene were added to the organic phase. The organic

solvent was removed in vacuo to produce a film. The film was resuspended in 10 ml benzene and the separation and solvent removal steps were repeated. The film was stored suspended in 25 ml of chloroform.

The digalactosyl diglyceride was purified from the above film suspension by the following procedure: Fifteen grams of salicic acid that had been activated by baking at 100 ° C for three hours was combined with 50 ml of chloroform. The salicic acid slurry was packed into a 20 cm x 40 cm column and the bed washed twice with chloroform. 175 g (5 ml) of the lipid solution was loaded onto the column and the flow rate adjusted to 3-5 ml per minute. 175 ml of chloroform was applied to the column, removing pigments in 12 ml fractions; followed by 70 ml of chloroform:acetone (1:1 v/v) applied to the column; followed by 700 ml of acetone. The first (5) 12 ml fractions contain MGDG, followed by DGDG in fractions 9-14, and finally phospholipids, in the remaining fractions which were discarded. Purity of the DGDG was assayed using thin layer chromatography ("TLC"), according to the procedures of Rouser, et al. (Lipid Chromatographic Analysis, Dekker Inc., New York, 1, pp. 99-162, 1967).

Fractions containing DGDG, as determined by TLC were combined and rotoevaporated under reduced pressure to a film. Chloroform (10 ml) was added and the solution transferred to a pre-weighed flask, and rotoevaporated under reduced pressure to a film. The flask containing lipid film was again weighed and the difference calculated to be the weight of the lipid.

The present invention is exemplified by the following Examples,

## EXAMPLE 1

### PREPARATION OF A MONSTEROIDAL ANTI-INFLAMMATORY DRUG COMPOSITION

Digalactosyl diglyceride (DGDG) (500 mg) (obtained from Serdary Research Laboratories, London, Ontario, Canada) in chloroform at 5 mg/ml was combined with 25 mg of indomethacin in a round bottom flask. The chloroform was removed by evaporation under reduced pressure. Diethyl ether (5 ml) was added to the resulting lipid-drug film and the film resuspended. The flask was placed in a bath sonicator and 1.0 ml of PBS pH 7.4 was added. The solvent was removed under a nitrogen stream while sonicating. The resulting lipid-drug paste was rehydrated with 2.3 ml PBS at pH 7.4. Resulting liposomes contained 10 mg/ml indomethacin.

## CONTROL 1

### INDUCING INTESTINAL ULCERATION (ACUTE)

Twenty 225-250 g male Wistar rats were starved for 18-24 hours prior to dosing. Rats were allowed access to water throughout the study. Ten rats in the free drug control group were then administered one oral dose of indomethacin, dissolved in polyethylene glycol 400 at 7 mg/ml and administered at 10 mg/kg body weight of indomethacin.

Four hours following dosing, rats were sacrificed by carbon dioxide anoxia and their stomachs surgically removed by severing at the cardiac and pyloric sphinctors. The stomachs were opened along the lesser curve, flattened, and washed with saline. Ulcer lengths were counted under a dissecting microscope (American Optical, Buffalo, NY) equipped with an eyepiece micrometer, and lengths were summed and averaged for all animals in a single treatment group.

Ulcer protection was assessed by summing and averaging ulcer lengths (in mm) of the treated group and comparing the value to that of the group administered free drug (Example 3). Percent ulcer inhibition was calculated by dividing the average length of ulceration of the liposome-treated group by the of the corresponding free drug group, and multiplying by 100.

## EXAMPLE 2

### ADMINISTRATION OF DGDG LIPOSOMES

The procedures and materials of Example 1 were employed using 10 mg/kg body weight of indomethacin entrapped in stable plurilamellar vesicles (SPLVs) composed of digalactosyl diglyceride; ulcer protection was assessed as in Example 1. Table 1 demonstrates that the oral administration of indomethacin in DGDG liposomes ameliorates the ulcerative activity of indomethacin, as compared to that of free indomethacin.

CONTROL 2

INFLAMMATION REDUCTION

Eight female Wistar rats weighing approximately 100 grams were allowed free access to food and water. A tatoo line was inscribed onto the right rear paw just below the hair line. Initial paw volume was measured by a transducer-linked plethysmometer (Stoelting Co., Chicago, Illinois) which contained a saline solution. The instrument measures paw volumes by an electrical charge difference across two electrodes resulting from the volume displacement by the paw. A transducer corrects this charge difference into cubic centimeters of volume displaced. Following immersion of the paw in the perspex cell to the inscribed line, a direct measurement of the displacement volume was recorded.

Rats were orally dosed with 2 mg/kg body weight of indomethacin in polyethylene glycol 400.

At 30 minutes post treatment, rats received an injection of 0.1 ml of 1.5% carageenan in saline (1.0M NaCl) directly into the rear paw pad. The paw volume was again determined 2.5 hours after the carageenan administration. The edema intensity (EI) was calculated:

$$\text{Edema Intensity} = \frac{\text{Final paw volume} - \text{initial paw volume}}{\text{Initial paw volume}}$$

and averaged for the eight rats.

The paw volumes for the eight rats were averaged and the percent swelling inhibition was calculated:

% swelling Reduction:

$$\frac{\text{Untreated control avg. EI} - \text{Treated group avg. EI}}{\text{Untreated control avg. EI}} \times 100$$

EXAMPLE 3

INFLAMMATION REDUCTION

The procedures and materials of Example 2 were employed using 2 mg/kg of indomethacin entrapped in SPLVs made with DGDG. Rats were orally dosed with the liposomes, and percent swelling reduction was calculated as in Example 2. Table 2 shows the comparable swelling reduction of free indomethacin (Example 2) and DGDG-liposome entrapped indomethacin in this acute carageenan paw edema model.

Example 4

LIPOSOME PREPARATION

Preparation of a nonsteroidal anti-inflammatory therapeutic agent comprising 200 mg of lipid was dissolved into 5 ml of ethanol. To that solution was added 10 mg of indomethacin. To the resulting solution 0.6 ml of saline was added and the solvent then was removed by rotoevaporation, leaving a film.

The film was resuspended thus forming liposomes at the desired concentration of indomethacin. For the liposomes of Control 3 the material was brought up to 2 ml final volume and for Control 4 the material was brought up to 5 ml.

Example 5

PREPARATION OF SPLV LIPOSOMES (STABLE VESICLES)

In a flask 18.48 gm of indomethacin and 369.6 gm egg phosphatidylcholine were dissolved in 1848 ml methylene chloride at room temperature and the sample pressure chloride. To that was added 1,108 ml saline. The lipid phase was removed while stirring under vacuum until solvent is removed. To the resulting material was added saline to a final volume of 3.186 litres. This preparation was then ready for administration.

Example 6

TEST PROCEDURE : LIPOSOMALLY ENTRAPPED/EXOGENOUS NONSTEROIDAL ANTI-INFLAMMATORY THERAPEUTIC AGENT

In this example, 0.25 uCi of $^{14}C$-indomethacin were included in 100 mg preparations of liposomes containing variously 15 to 25 mg of indomethacin prepared by the method of Example 4. The aqueous phase of the liposomes were comprised of 0.3 ml aliquots of Ringer saline solution containing 10 mM of calcium. These aliquots were then loaded onto continuous 10 ml, 5-20% sucrose gradients and centrifuged at 288,000 xg for 2.5 hours. The specific results of this test are shown in Figure 1. The specific conditions for determining percentages of exogenous nonsteroidal anti-inflammatory therapeutic agent by this method or by other methods as well known to those skilled in the art.

Example 7

WASHING LIPOSOMAL PREPARATIONS

The liposome preparation of Example 6 washed 3 times with 5 ml saline after one hour of centrifugation at 25,000 g retains less than 5% exogenous indomethacin.

Control 3

INDUCING GASTRIC ULCERS (ACUTE)

Indomethacin at dosages between about 2 mg/kg and 10 mg/kg were given in 0.5 ml of neat PEG-400, vehicle or as incorporated into the liposomes at a 200 mg/kg dosage of phospholipid. Four hours following oral administration of test formulations, animals were sacrificed by $CO_2$ anoxia and the stomachs were excised. For systemically-induced gastric ulceration, dosages of PEG-400 solubilized indomethacin between 4 mg/kg and 50 mg/kg in 0.5 ml were administered subcutaneously. For subsequent experiments a dosage of 30 mg/kg was found to induce the maximum response. Tissue was thoroughly rinsed in saline and the inner mucosal surface was laid flat for microscopic evaluation. Visible ulcers were quantitated using a dissecting microscope (AO Optical, Buffalo, NY) equipped with an eyepiece micrometer. Results using this test were expressed in millimeters of ulceration and in percent inhibition of ulcers where treatment was effected. For all studies, a minimum of 10 animals per experimental group were employed. Experiments were conducted in triplicate, thereby representing a minimum of 30 rats per group.

Control 4

INDUCING INTESTINAL ULCERATION (CHRONIC)

Single daily oral dosages of indomethacin at about 4 mg/kg in vehicle or in monophasic vesicles were administered over 4 days. Test animals in this example were rats (Wistar). On the fifth day the full length of the intestines were excised and slit open opposite to the attached mesenteric issue. The mucosal surface was rinsed with saline, the ulcers (including perforations) were quantitated in millimeters as described about or by determination of total surface area of lesions. This was accomplished by means of a planimeter (Zidas, Carl Zeiss, West Germany) optically linked to an SR stereo dissecting microscope (Zeiss) with a draw tube attachment. In this example results were expressed as total $mm^2$ of ulcerated surface and as percent inhibition of ulceration when treatment was provided. Average values were calculated based upon a

minimum of 5 animals per group from at least duplicate experiments. A higher mortality rate (20-30%) was observed in rats receiving indomethacin solubilized in vehicles at this dosage then in liposome-treated groups. Sham vehicle, however, were nontoxic to the animals. Clearly this method is easily adaptable by those skilled in the art to other nonsteroidal anti-inflammatory therapeutic agents and other animals.

Example 8

PREPARATION OF AND DRUG RELEASE WITH HYDROGENATED LIPOSOMES

Three formulations for comparison of saturated and unsaturated liposomes were prepared by the method of Example 5, each containing indomethacin as the therapeutic agent. These were liposomes made from (a) 5 gm of EPC and 100 mg of indomethacin, (b) 5 gm of hydrogenated soy phosphatidylcholine and 100 mg of indomethacin, and (c) 3.35 gm hydrogenated soy phosphatidylcholine, 1.645 gm cholesterol and, 100 mg indomethacin.

The resulting liposomes were administered orally to rats at a dosage of 10 mg/kg and blood levels of indomethacin determined over a 24 hour period by high pressure liquid chromatography. The results shown in FIG 2 discloses a distinct blood level profile for release of nonsteroidal anti-inflammatory therapeutic agent into the blood after oral administration in gastric resistant liposomes. The hydrogenated soy phosphatidylcholine displays a "controlled release" absent the plasma peak characteristic of other liposomes of less saturation. This pattern of release indicates that some liposomes will be excreted prior to delivery of all therapeutic agent which must be taken into account in selecting the dosage to be administered to yield the dosage taken up from the gastrointestinal tract.

It is to be particularly understood that any nonsteroidal anti-inflammatory, alone or in combination with others, may be used as primarily encapsulated in any gasric resistant liposome.

TABLE 1

| TREATMENT | INDOMETHACIN DOSAGE (mg/kg) | mm ULCERATION | % ULCER INHIBITION |
|---|---|---|---|
| Free Indomethacin in Vehicle | 10 | 43.9 | - |
| Indomethacin-DGDG SPLVs | 10 | 1.1 | 97.5 |

TABLE 2

| TREATMENT | IDOMETHACIN DOSAGE (mg/kg) | % SWELLING REDUCTION |
|---|---|---|
| Free Indomethacin | 2 | 37.2 |
| Indomethacin-DGDG-SPLV | 2 | 36.9 |

TABLE 3

| COMPARISON OF GASTRIC ULCERATION PRODUCED FROM ACUTE ORAL ADMINISTRATION OF INDOMETHACIN IN VEHICLES OR LIPOSOMES | | | |
|---|---|---|---|
| Treatment | Indomethacin Dosage (mg/kg) | mm Ulceration + SEM* | % Ulcer Inhibition Relative to PEG-400 Control |
| Indomethacin in | 2 | 1.1 ± 0.3 | - |
| PEG-400 | 4<br>6<br>8<br>10 | 3.4 ± 0.8<br>9.7 ± 1.2<br>22.3 ± 3.4<br>23.3 ± 2.6 | -<br>-<br>-<br>- |
| Indomethacin-EPC Liposomes | 2<br>4<br>6<br>8<br>10 | 0<br>0.8 ± 0.3<br>1.8 ± 0.6<br>3.3 ± 0.9<br>3.6 ± 1.1 | 100<br>76.4<br>81.4<br>85.2<br>85.7 |
| Indomethacin-$CHS_t$ Liposomes<br>Indomethacin-$THS_t$ | 10<br>10 | 28.7 ± 4.8<br>19.4 ± 4.4 | (-)13.4<br>23.3 |

*Standard error of the mean

TABLE 4

| C0MPARISON OF INTESTINAL ULCERATION FOLLOWING 4-DAY CHRONIC ORAL DOSING OF INDOMETHACIN IN EITHER VEHICLES OR LIPOSOMES | | | |
|---|---|---|---|
| Vehicle | Indomethacin Dosage (mg/kg) | $mm^2$ Ulceration + SEM | % Ulcer Inhibition Relative to PEG-400 Control |
| PEG-400 | 4 | 759.3 ± 120.6 | |
| EPC-Lipsomes | 4 | 91.4 ± 35.4 | 86.1 |
| $CHS_t$ Liposomes | 4 | 1084.20 ± 160.6 | (-)64.5 |
| $THS_t$ Liposomes | 4 | 398.6 ± 230.7 | 39.5 |
| HSPC Liposomes | 4 | 80.7 ± 42.8 | 87.7 |

## Claims

**Claims for the following Contracting States : BE, CH, DE, FR, GB, IT, LI, LU, NL, SE**

**1.** A liposomal composition comprising a therapeutically effective amount of at least one nonsteroidal anti-inflammatory therapeutic agent primarily encapsulated in a gastric resistant liposome and primarily absent exogenous nonsteroidal anti-inflammatory therapeutic agent optionally the composition additionally comprises a pharmaceutically acceptable carrier or diluent.

**2.** The composition of claim 1 wherein said exogenous nonsteroidal anti-inflammatory therapeutic agent comprises less than about 30% by weight of nonsteroidal anti-inflammatory drug present and preferably less than about 5%.

**3.** The composition of claims 1 or 2 wherein the gastric resistant liposome is substantially comprised of a saturated lipid, preferably hydrogenated egg or soy phosphatidylcholine.

**4.** The composition of claims 1 or 2 wherein the gastric resistant liposome comprises egg phosphatidylcholine.

5. The composition of claims 1 or 2 wherein the gastric resistant liposome comprises a glycolipid, preferably a glycosphingolipid or a galactolipid.

6. The composition of claim 4 wherein the galactolipid is digalactosyl diglyceride or monogalactosyl diglyceride.

7. The composition of any of the foregoing claims comprising a phospholipid, a sterol derivative, or a tocopherol derivative.

8. The composition of any of the foregoing claims wherein the gastric resistant liposome is of substantially equal solute distribution preferably stable plurilamellarvesicle (SPLV) monophasic vesicle (MPV) or multilamellarvesicle produced by a freeze and show thaw technique (FATMLV).

9. The composition of claims 7 or 8 wherein the nonsteroidal anti-inflammatory drug is selected from the group consisting of acemetacin, alclofenac, azapropazone, benoxaprofen, benrilate, carprofen, cholinemagnesium trisalicylate, diclofenac, diflunisal, etodolac, fenbufen, fenclofenac, fenoprofen, fentiazac, feprazone, flufenamino acid, flurbiprofen, glucametacin, ibuprofen, indomethacin, indoprofen, isoxicam, ketoprofen, magnesium salicylic acid, meclofenamic acid, mefenamino acid, methylsalicylate, naproxen, niflumic acid, osmosin, oxaprozin, oxphenbutazone, phenylbutazone, piroxicam, pirprofen, salicylamide, salicylic acid, salicylic acid acetate, salicylsalicylic acid, sodium salicylate, sulindac, suprofen, tenoxicam, tiaprofenic acid, tolmetin, and zomepirac.

10. The composition of any of the foregoing claims wherein the nonsteroidal anti-inflammatory therapeutic agent is indomethacin.

11. The use of the composition of any of the foregoing claims in the manufacture of a pharmaceutical for minimizing the gastrointestinal irritation associated with treatment by the administration of a therapeutically effective dose of at least one nonsteroidal anti-inflammatory drug to mammal including a human.

12. The use of the composition of any of the foregoing claims in the manufacture of a pharmaceutical to treat inflammation, pain or fever in a mammal by administering an anti-inflammatory effective amount, an analgesic effective amount or anti-pyretic amount.

13. A method of preparing gastric resistant liposomes according to any one of the foregoing claims comprising
   (a) forming a solution of at least one gastric resistant lipid in at least one organic solvent plus a first aqueous component containing therein at least one nonsteroidal anti-inflammatory drug in amounts sufficient to form a monophase;
   (b) evaporating organic solvent of the monophase at a temperature and pressure which maintains the monophase and facilitates evaporation until a film forms;
   (c) adding a second aqueous component to the film and agitating the second aqueous component with the film in order to resuspend the film and to form lipid vesicles encapsulating said nonsteroidal anti-inflammatory drug; and
   (d) preferably treating the product of step (c) by removing excess exogenous nonsteroidal anti-inflammatory drug preferably by washing in a aqueous solution.

**Claims for the following Contracting States : AT, ES, GR**

1. A method for preparing liposomal composition comprising encapsulating a therapeutically effective amount of at least one nonsteroidal anti-inflammatory therapeutic agent primarily in a gastric resistant liposome and primarily absent exogenous nonsteroidal anti-inflammatory therapeutic agent and optionally adding a pharmaceutically acceptable carrier or diluent.

2. The method of claim 1 wherein said exogenous nonsteroidal anti-inflammatory therapeutic agent comprises less than about 30% by weight of nonsteroidal anti-inflammatory drug present and preferably less than about 5%.

3. The method of claims 1 or 2 wherein the gastric resistant liposome is substantially comprised of a saturated lipid, preferably hydrogenated egg or soy phosphatidylcholine.

4. The method of claims 1 or 2 wherein the gastric resistant liposome comprises egg phosphatidylcholine.

5. The method of claims 1 or 2 wherein the gastric resistant liposome comprises a glycolipid, preferably a glycosphingolipid or a galactolipid.

6. The method of claim 4 wherein the galactolipid is digalactosyl diglyceride or monogalactosyl diglyceride.

7. The method of any of the foregoing claims comprising a phospholipid, a sterol derivative, or a tocopherol derivative.

8. The method of any of the foregoing claims wherein the gastric resistant liposome is of substantially equal solute distribution preferably stable plurilamellarvesicle (SPLV) monophasic vesicle (MPV) or multilamellarvesicle produced by a freeze and show thaw technique (FATMLV).

9. The method of claims 7 or 8 wherein the nonsteroidal anti-inflammatory drug is selected from the group consisting of acemetacin, alclofenac, azapropazone, benoxaprofen, benrilate, carprofen, cholinemagnesium trisalicylate, diclofenac, diflunisal, etodolac, fenbufen, fenclofenac, fenoprofen, fentiazac, feprazone, flufenamino acid, flurbiprofen, glucametacin, ibuprofen, indomethacin, indoprofen, isoxicam, ketoprofen, magnesium salicylic acid, meclofenamic acid, mefenamino acid, methylsalicylate, naproxen, niflumic acid, osmosin, oxaprozin, oxphenbutazone, phenylbutazone, piroxicam, pirprofen, salicylamide, salicylic acid, salicylic acid acetate, salicylsalicylic acid, sodium salicylate, sulindac, suprofen, tenoxicam, tiaprofenic acid, tolmetin, and zomepirac.

10. The method of any of the foregoing claims wherein the nonsteroidal anti-inflammatory therapeutic agent is indomethacin.

11. The use of the composition of any of the foregoing claims in the manufacture of a pharmaceutical for minimizing the gastrointestinal irritation associated with treatment by the administration of a therapeutically effective dose of at least one nonsteroidal anti-inflammatory drug to mammal including a human.

12. The use of the composition of any of the foregoing claims in the manufacture of a pharmaceutical to treat inflammation, pain or fever in a mammal by administering an anti-inflammatory effective amount, an analgesic effective amount or anti-pyretic amount.

13. A method of preparing gastric resistant liposomes according to any one of the foregoing claims comprising
(a) forming a solution of at least one gastric resistant lipid in at least one organic solvent plus a first aqueous component containing therein at least one nonsteroidal anti-inflammatory drug in amounts sufficient to form a monophase;
(b) evaporating organic solvent of the monophase at a temperature and pressure which maintains the monophase and facilitates evaporation until a film forms;
(c) adding a second aqueous component to the film and agitating the second aqueous component with the film in order to resuspend the film and to form lipid vesicles encapsulating said nonsteroidal anti-inflammatory drug; and
(d) preferably treating the product of step (c) by removing excess exogenous nonsteroidal anti-inflammatory drug preferably by washing in a aqueous solution.

## Revendications

### Revendications pour les Etats contractants suivants : BE, CH, DE, FR, GB, IT, LI, LU, NL, SE

1. Composition liposomique comprenant une quantité thérapeutiquement efficace d'au moins un agent thérapeutique anti-inflammatoire non-stéroïdique essentiellement encapsulé dans un liposome résistant au suc gastrique et essentiellement débarrassée de la présence d'un agent thérapeutique anti-inflammatoire non-stéroïdique exogène, la composition comprenant en outre si on le désire un support ou diluant pharmaceutiquement acceptable.

2. Composition de la revendication 1 dans laquelle ledit agent thérapeutique anti-inflammatoire non-stéroïdique exogène constitue moins d'environ 30% en poids du médicament anti-inflammatoire non-stéroïdique présent, et de préférence moins d'environ 5%.

3. Composition des revendications 1 ou 2 dans laquelle le liposome résistant au suc gastrique est pour l'essentiel composé d'un lipide saturé, de préférence la phosphatidylcholine d'oeuf ou de soja hydrogénée.

4. Composition des revendications 1 ou 2 dans laquelle le liposome résistant au suc gastrique comprend de la phosphatidylcholine d'oeuf.

5. Composition des revendications 1 ou 2 dans laquelle le liposome résistant au suc gastrique comprend un glycolipide, de préférence un glycosphingolipide ou un galactolipide.

6. Composition de la revendication 4 dans laquelle le galactolipide est le digalactosyl-glycéride ou le monogalactosyl-diglycéride.

7. Composition de l'une quelconque des revendications précédentes comprenant un phospholipide, un dérivé de stérol ou un dérivé de tocophérol.

8. Composition de l'une quelconque des revendications précédentes dans laquelle le liposome résistant au suc gastrique a une distribution sensiblement égale dans le soluté de préférence stable entre les vésicules plurilamellaires (SPLV), les vésicules monophasiques (MPV) ou les vésicules multilamellaires produites par une technique de congélation et décongélation (FATMLV).

9. Composition des revendications 7 ou 8 dans laquelle le médicament anti-inflammatoire non-stéroïdique est choisi dans le groupe constitué par l'acémétacine, l'aclofénac, l'azapropazone, le benoxaprofène, le benrilate, le carprofène, le trisalicylate de cholinemagnésium, le diclofénac, le diflunisal, l'étodolac, le fenbufène, le fenclofénac, le fénoprofène, le fentiazac, la féprazone, l'acide flufénaminé, le flurbiprofène, la glucamétacine, l'ibuprofène, l'indométhacine, l'indoprofène, l'isoxicam, le kétoprofène, l'acide magnésium-salicylique, l'acide méclofénamique, l'acide méfénaminé, le salicylate de méthyle, le naproxène, l'acide niflumique, l'osmosine, l'oxaprozine, l'oxphenbutazone, la phénylbutazone, le piroxicam, le pirprofène, le salicylamide, l'acide salicylique, l'acétate de l'acide salicylique, l'acide salicylsalicylique, le salicylate de sodium, le sulindac, le suprofène, le tenoxicam, l'acide tiaprofénique, la tolmétine et le zomépirac.

10. Composition de l'une quelconque des revendications précédentes dans laquelle l'agent thérapeutique anti-inflammatoire non-stéroïdique est l'indométhacine.

11. Application de la composition de l'une quelconque des revendications précédentes à la préparation d'un médicament pour réduire au minimum l'irritation gastrointestinale associée au traitement par l'administration d'une dose thérapeutiquement efficace d'au moins un médicament anti-inflammatoire non-stéroïdique à un mammifère y compris l'homme.

12. Application d'une composition de l'une quelconque des revendications précédentes à la préparation d'un médicament pour traiter l'inflammation, la douleur ou la fièvre chez un mammifère en en administrant une quantité efficace comme anti-inflammatoire, une quantité efficace comme analgésique ou une quantité anti-pyrétique.

**13.** Procédé de préparation de liposomes résistant au suc gastrique selon l'une quelconque des revendications précédentes, dans lequel

(a) on forme une solution d'au moins un lipide résistant au suc gastrique dans au moins un solvant organique additionné d'un premier composant aqueux contenant au moins un médicament anti-inflammatoire non-stéroïdique en quantités suffisantes pour former une monophase;

(b) on fait évaporer le solvant organique de la monophase à une température et à une pression qui maintiennent la monophase et qui facilitent l'évaporation jusqu'à formation d'une pellicule;

(c) on ajoute un second composant aqueux à la pellicule et on agite le second composant aqueux avec la pellicule afin de remettre en suspension la pellicule et de former des vésicules lipides encapsulant ledit médicament anti-inflammatoire non-stéroïdique; et

(d) de préférence on traite le produit de l'étape (c) en enlevant l'excès de médicament anti-inflammatoire non-stéroïdique exogène de préférence par lavage dans une solution aqueuse.

**Revendications pour les Etats contractants suivants : AT, ES, GR**

**1.** Procédé de préparation d'une composition liposomique dans lequel on encapsule une quantité thérapeutiquement efficace d'au moins un agent thérapeutique anti-inflammatoire non-stéroïdique essentiellement dans un liposome résistant au suc gastrique et sensiblement en l'absence d'agent thérapeutique anti-inflammatoire non-stéroïdique exogène et si on le désire on ajoute un support ou diluant pharmaceutiquement acceptable.

**2.** Procédé de la revendication 1 dans lequel ledit agent thérapeutique anti-inflammatoire non-stéroïdique exogène constitue moins d'environ 30% en poids de médicament anti-inflammatoire non-stéroïdique présent et de préférence moins d'environ 5%.

**3.** Procédé des revendications 1 ou 2 dans lequel le liposome résistant au suc gastrique est pour l'essentiel composé d'un lipide saturé, de préférence de la phosphatidylcholine d'oeuf ou de soja hydrogénée.

**4.** Procédé des revendications 1 ou 2 dans lequel le liposome résistant au suc gastrique comprend de la phosphatidylcholine d'oeuf.

**5.** Procédé des revendications 1 ou 2 dans lequel le liposome résistant au suc gastrique comprend un glycolipide, de préférence un glycosphingolipide ou un galactolipide.

**6.** Procédé de la revendication 4 dans lequel le galactolipide est le digalactosyl-diglycéride ou le monogalactosyl-diglycéride.

**7.** Procédé de l'une quelconque des revendications précédentes comprenantun phospholipide, un dérivé de stérol ou un dérivé de tocophérol.

**8.** Procédé de l'une quelconque des revendications précédentes dans lequel le liposome résistant au suc gastrique a une distribution sensiblement égale dans le soluté de préférence stable entre les vésicules plurilamellaires (SPLV), les vésicules monophasiques (MPV) ou les vésicules multilamellaires produites par une technique de congélation et décongélation manifeste (FATMLV).

**9.** Procédé des revendications 6 ou 8 dans lequel le médicament anti-inflammatoire non-stéroïdique est choisi dans le groupe constitué par l'acémétacine, l'alclofénac, l'azapropazone, le bénoxaprofène, le benrilate, le carprofène, le trisalicylate de cholinemagnésium, le diclofénac, le diflunisal, l'étodolac, le fenbufène, le fenclofénac, le fénoprofène, le fentiazac, la féprazone, l'acide flufénaminé, le flurbiprofène, la glucamétacine, l'ibuprofène, l'indométhacine, l'indoprofène, l'isoxicam, le kétoprofène, l'acide magnésium-salicylique, l'acide méclofénamique, l'acide méfénaminé, le salicylate de méthyle, le naproxène, l'acide niflumique, l'osmosine, l'oxaprozine, l'oxphenbutazone, la phénylbutazone, le piroxicam, le pirprofène, le salicylamide, l'acide salicylique, l'acétate de l'acide salicylique, l'acide salicylsalicylique, le salicylate de sodium, le sulindac, le suprofène, le tenoxicam, l'acide tiaprofénique, la tolmétine et le zomépirac.

10. Procédé de l'une quelconque des revendications précédentes dans lequel l'agent thérapeutique anti-inflammatoire non-stéroïdique est l'indométhacine.

11. Application de la composition de l'une quelconque des revendications précédentes à la préparation d'un médicament pour réduire au minimum l'irritation gastro-intestinale associée au traitement par l'administration d'une dose thérapeutiquement efficace d'au moins un médicament anti-inflammatoire non-stéroïdique à un mammifère y compris l'homme.

12. Application de la composition de l'une quelconque des revendications précédentes à la préparation d'un médicament pour traiter l'inflammation, la douleur ou la fièvre chez un mammifère en administrant une quantité efficace comme anti-inflammatoire, une quantité effiface comme analgésique ou une quantité antipyrétique.

13. Procédé de préparation de liposomes résistant au suc gastrique selon l'une quelconque des revendications précédentes dans lequel
(a) on forme une solution d'au moins un lipide résistant au suc gastrique dans au moins un solvant organique additionné d'un premier composant aqueux contenant au moins un médicament anti-inflammatoire non-stéroïdique en des quantités suffisantes pour former une monophase;
(b) on fait évaporer le solvant organique de la monophase à une température et à une pression qui maintiennent la monophase et facilitent l'évaporation jusqu'à formation d'une pellicule;
(c) on ajoute un second composant aqueux à la pellicule et on agite le second composant aqueux avec la pellicule afin de remettre en suspension la pellicule et de former des vésicules lipidiques encapsulant ledit médicament anti-inflammatoire non-stéroïdique; et
(d) de préférence on traite le produit de l'étape (c) en enlevant l'excès de médicament anti-inflammatoire non-stéroïdique exogène de préférence par lavage dans une solution aqueuse.

**Patentansprüche**

**Patentansprüche für folgende Vertragsstaaten : BE, CH, DE, FR, GB, IT, LI, LU, NL, SE**

1. Liposomzusammensetzung, umfassend eine therapeutisch wirksame Menge mindestens eines nichtsteroiden antiinflammatorischen therapeutischen Mittels, vorwiegend eingekapselt in ein magenbeständiges Liposom und vorwiegend ohne exogenes nichtsteroides antiinflammatorisches therapeutisches Mittel, wobei die Zusammensetzung gegebenenfalls ein(en) pharmazeutisch brauchbar(es)(en) Träger oder Verdünnungsmittel umfaßt.

2. Zusammensetzung nach Anspruch 1, worin das exogene nichtsteroide antiinflammatorische therapeutische Mittel weniger als etwa 30 Gew.-% des vorhandenen nichtsteroiden antiinflammatorischen Arzneimittels und vorzugsweise weniger als etwa 5 % umfaßt.

3. Zusammensetzung nach Anspruch 1 oder 2, worin das magenbeständige Liposom im wesentlichen ein gesättigtes Lipid, vorzugsweise hydriertes Ei- oder Soja-phosphatidylcholin, umfaßt.

4. Zusammensetzung nach den Ansprüchen 1 oder 2, worin das magenbeständige Liposom Ei-phosphatidylcholin umfaßt.

5. Zusammensetzung nach Anspruch 1 oder 2, worin das magenbeständige Liposom ein Glykolipid, vorzugsweise ein Glykosphingolipid oder ein Galactolipid umfaßt.

6. Zusammensetzung nach Anspruch 4, worin das Galactolipid Digalactosyl-diglycerid oder Monogalactosyl-diglycerid ist.

7. Zusammensetzung nach einem der vorhergehenden Ansprüche, umfassend ein Phospholipid, ein Sterinderivat oder ein Tocopherolderivat.

**8.** Zusammensetzung nach einem der vorhergehenden Ansprüche, worin das magenbeständige Liposom im wesentlichen gleiche Lösungsverteilung hat und vorzugsweise stabile plurilamellare Vesikel (SPLV), monophasische Vesikel (MPV) oder multilamellare Vesikel, hergestellt nach einer Gefrier- und Auftau-Technik (FATMLV) darstellt.

**9.** Zusammensetzung nach den Ansprüchen 7 oder 8, worin das nichtsteroide antiinflammatorische Arzneimittel ausgewählt ist aus der Gruppe von Acemetacin, Alclofenac, Azapropazon, Benoxaprofen, Benrilat, Carprofen, Cholinmagnesium-trisalicylat, Diclofenac, Diflunisal, Etodolac, Fenbufen, Fenclofenac, Fenoprofen, Fentiazac, Feprazon, Flufenaminosäure, Flurbiprofen, Glucametacin, Ibuprofen, Indomethacin, Indoprofen, Isoxicam, Ketoprofen, Magnesium-salicylsäure, Meclofenaminsäure, Mefenaminosäure, Methylsalicylat, Naproxen, Nifluminsäure, Osmosin, Oxaprozin, Oxphenbutazon, Phenylbutazon, Piroxicam, Pirprofen, Salicylamid, Salicylsäure, Salicylsäure-acetat, Salicylsalicylsäure, Natriumsalicylat, Sulindac, Suprofen, Tenoxicam, Tiaprofensäure, Tolmetin und Zomepirac.

**10.** Zusammensetzung nach einem der vorhergehenden Ansprüche, worin das nichtsteroide antiinflammatorische therapeutische Mittel Indomethacin ist.

**11.** Verwendung der Zusammensetzung nach einem der vorhergehenden Ansprüche bei der Herstellung eines pharmazeutischen Mittels zur Verringerung der gastrointestinalen Reizung, die mit der Behandlung durch Verabreichung einer therapeutisch wirksamen Dosis mindestens eines nichtsteroiden antiinflammatorischen Arzneimittels an Säuger, einschließlich den Menschen, einhergeht.

**12.** Verwendung der Zusammensetzung nach einem der vorhergehenden Ansprüche bei der Herstellung eines pharmazeutischen Mittels zur Behandlung von Entzündungen, Schmerzen oder Fieber eines Säugers, durch Verabreichung einer antiflammatorisch wirksamen Menge, einer analgetisch wirksamen Menge oder antipyretischen Menge.

**13.** Verfahren zur Herstellung von magenresistenten Liposomen nach einem der vorhergehenden Ansprüche, umfassend

(a) die Bildung einer Lösung von mindestens einem magenresistenten Lipid in mindestens einem organischen Lösungsmittel plus einer ersten wäßrigen Komponente, die mindestens ein nichtsteroides antiinflammatorisches Arzneimittel enthält, in ausreichenden Mengen zur Bildung einer Monophase;

(b) das Verdampfen des organischen Lösungsmittels aus der Monophase bei einer Temperatur und einem Druck, der die Monophase aufrecht erhält und die Verdampfung bis zur Bildung eines Filmes erleichtert;

(c) den Zusatz einer zweiten wäßrigen Komponente zu dem Film und Bewegen der zweiten wäßrigen Komponente mit dem Film, um den Film erneut zu suspendieren und Lipidviskel zu bilden, die das nichtsteroide antiinflammatorische Arzneimittel einkapseln; und

(d) vorzugsweise das Behandeln des Produkts der Stufe (c) durch Entfernen von überschüssigem exogenen nichtsteroidem antiinflammatorischem Arzneimittel, vorzugsweise durch Waschen in einer wäßrigen Lösung.

**Patentansprüche für folgende Vertragsstaaten : AT, ES, GR**

**1.** Verfahren zur Herstellung einer Liposomzusammensetzung, umfassend die Einkapselung einer therapeutisch wirksamen Menge mindestens eines nichtsteroiden antiinflammatorischen therapeutischen Mittels, vorwiegend in ein magenbeständiges Liposom und vorwiegend ohne exogenes nichtsteroides antiinflammatorisches therapeutisches Mittel, und gegebenenfalls den Zusatz eines pharmazeutisch brauchbaren Trägers oder Verdünnungsmittels.

**2.** Verfahren nach Anspruch 1, worin das exogene nichtsteroide antiinflammatorische therapeutische Mittel weniger als etwa 30 Gew.-% des vorhandenen nichtsteroiden antiinflammatorischen Arzneimittels und vorzugsweise weniger als etwa 5 % umfaßt.

**3.** Verfahren nach Anspruch 1 oder 2, worin das magenbeständige Liposom im wesentlichen ein gesättigtes Lipid, vorzugsweise hydriertes Ei- oder Soja-phosphatidylcholin, umfaßt.

4. Verfahren nach den Ansprüchen 1 oder 2, worin das magenbeständige Liposom Ei-phosphatidylcholin umfaßt.

5. Verfahren nach Anspruch 1 oder 2, worin das magenbeständige Liposom ein Glykolipid, vorzugsweise ein Glykosphingolipid oder ein Galactolipid umfaßt.

6. Verfahren nach Anspruch 4, worin das Galactolipid Digalactosyl-diglycerid oder Monogalactosyl-diglycerid ist.

7. Verfahren nach einem der vorhergehenden Ansprüche, umfassend ein Phospholipid, ein Sterinderivat oder ein Tocopherolderivat.

8. Verfahren nach einem der vorhergehenden Ansprüche, worin das magenbeständige Liposom im wesentlichen gleiche Lösungsverteilung hat und vorzugsweise stabile plurilamellare Vesikel (SPLV), monophasische Vesikel (MPV) oder multilamellare Vesikel, hergestellt nach einer Gefrier- und Auftau-Technik (FATMLV) darstellt.

9. Verfahren nach den Ansprüchen 7 oder 8, worin das nichtsteroide antiinflammatorische Arzneimittel ausgewählt ist aus der Gruppe von Acemetacin, Alclofenac, Azapropazon, Benoxaprofen, Benrilat, Carprofen, Cholinmagnesium-trisalicylat, Diclofenac, Diflunisal, Etodolac, Fenbufen, Fenclofenac, Fenoprofen, Fentiazac, Feprazon, Flufenaminosäure, Flurbiprofen, Glucametacin, Ibuprofen, Indomethacin, Indoprofen, Isoxicam, Ketoprofen, Magnesium-salicylsäure, Meclofenaminsäure, Mefenaminosäure, Methylsalicylat, Naproxen, Nifluminsäure, Osmosin, Oxaprozin, Oxphenbutazon, Phenylbutazon, Piroxicam, Pirprofen, Salicylamid, Salicylsäure, Salicylsäure-acetat, Salicylsalicylsäure, Natriumsalicylat, Sulindac, Suprofen, Tenoxicam, Tiaprofensäure, Tolmetin und Zomepirac.

10. Verfahren nach einem der vorhergehenden Ansprüche, worin das nichtsteroide antiinflammatorische therapeutische Mittel Indomethacin ist.

11. Verwendung der Zusammensetzung nach einem der vorhergehenden Ansprüche bei der Herstellung eines pharmazeutischen Mittels zur Verringerung der gastrointestinalen Reizung, die mit der Behandlung durch Verabreichung einer therapeutisch wirksamen Dosis mindestens eines nichtsteroiden antiinflammatorischen Arzneimittels an Säuger, einschließlich den Menschen, einhergeht.

12. Verwendung der Zusammensetzung nach einem der vorhergehenden Ansprüche bei der Herstellung eines pharmazeutischen Mittels zur Behandlung von Entzündungen, Schmerzen oder Fieber eines Säugers, durch Verabreichung einer antiflammatorisch wirksamen Menge, einer analgetisch wirksamen Menge oder antipyretischen Menge.

13. Verfahren zur Herstellung von magenresistenten Liposomen nach einem der vorhergehenden Ansprüche, umfassend
(a) die Bildung einer Lösung von mindestens einem magenresistenten Lipid in mindestens einem organischen Lösungsmittel plus einer ersten wäßrigen Komponente, die mindestens ein nichtsteroides antiinflammatorisches Arzneimittel enthält, in ausreichenden Mengen zur Bildung einer Monophase;
(b) das Verdampfen des organischen Lösungsmittels aus der Monophase bei einer Temperatur und einem Druck, der die Monophase aufrecht erhält und die Verdampfung bis zur Bildung eines Filmes erleichtert;
(c) den Zusatz einer zweiten wäßrigen Komponente zu dem Film und Bewegen der zweiten wäßrigen Komponente mit dem Film, um den Film erneut zu suspendieren und Lipidviskel zu bilden, die das nichtsteroide antiinflammatorische Arzneimittel einkapseln; und
(d) vorzugsweise das Behandeln des Produkts der Stufe (c) durch Entfernen von überschüssigem exogenem nichtsteroidem antiinflammatorischem Arzneimittel, vorzugsweise durch Waschen in einer wäßrigen Lösung.

FIG. 1

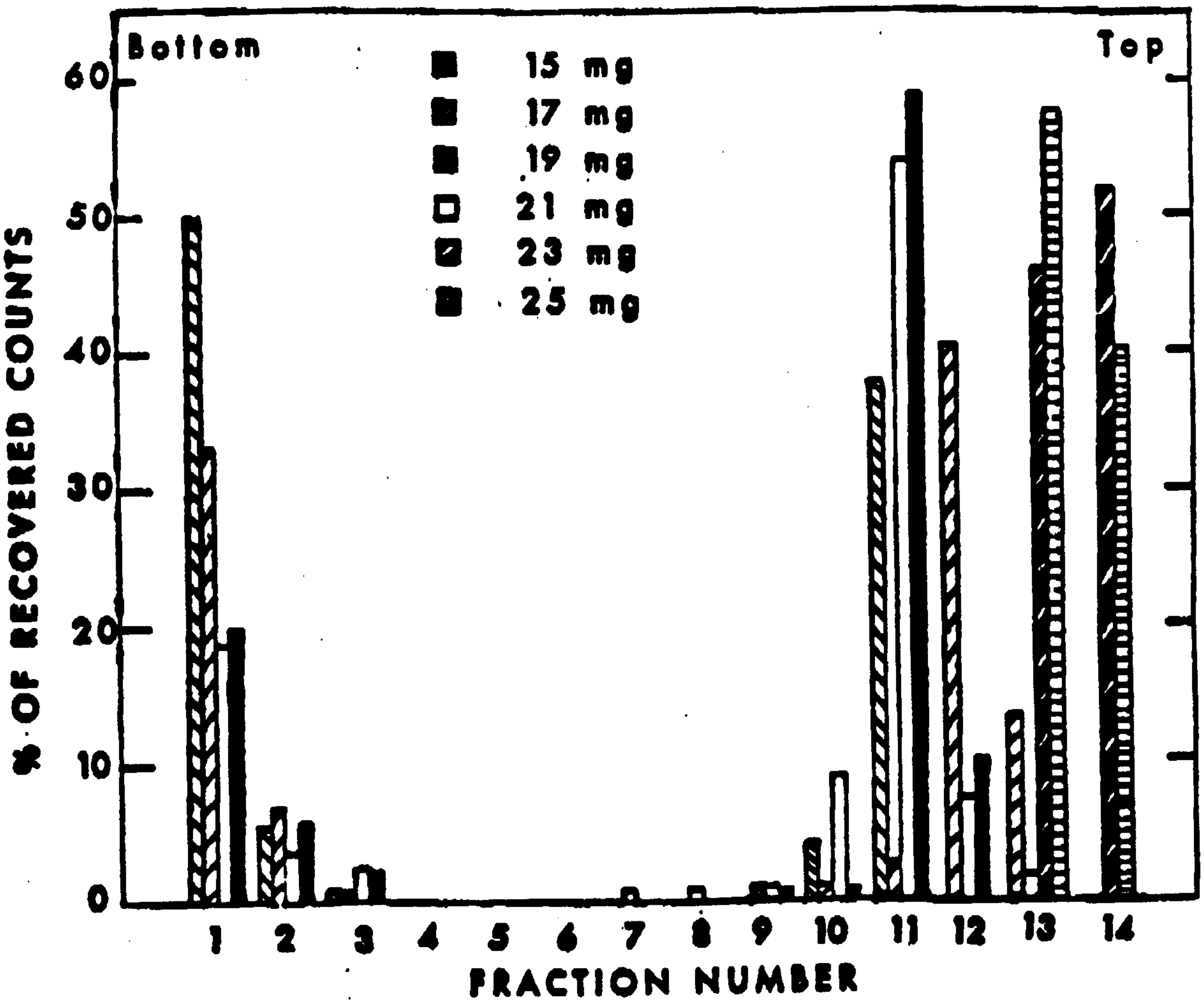
Bottom
Top
15 mg
17 mg
19 mg
21 mg
23 mg
25 mg
% OF RECOVERED COUNTS
0
10
20
30
40
50
60
1
2
3
4
5
6
7
8
9
10
11
12
13
14
FRACTION NUMBER

AVERASE INDOMETHACIN LEVELS ( ug/ml in PLASMA )

FIG. 2